# EUROPEAN PATENT SPECIFICATION

(11) **EP 1 197 235 B1**
(45) Date of publication and mention of the grant of the patent: **08.11.2006**
(21) Application number: 00931288.5
(22) Date of filing: 24.05.2000
(51) Int. Cl.: A61M 1/10, A61M 1/36, A61M 1/14

(54) **BLOOD PUMPING APPARATUS FOR EXTRACORPOREAL CIRCULATION AND VENTRICULAR ASSISTANCE**
BLUTPUMP VORRICHTUNG FÜR EINEN EXTRAKORPORALEN KREISLAUF UND ZUR HERZUNTERSTÜTZUNG
DISPOSITIF DE POMPAGE DU SANG POUR CIRCULATION EXTRACORPORELLE ET ASSISTANCE VENTRICULAIRE

(43) Date of publication of application: 17.04.2002
(73) Proprietor: Merce Vives, Salvador, 46004 Valencia (ES)
(72) Inventor: Merce Vives, Salvador, 46004 Valencia (ES)
(74) Representative: Ungria Lopez, Javier
(86) International application number: PCT/ES2000/000186
(87) International publication number: WO 2001/089600

(56) References cited:
- WO-A-92/04060
- ES-A- 2 011 147
- ES-A- 2 146 549
- US-A- 4 490 331
- US-A- 5 270 005
- US-A- 5 770 149
- US-A- 5 823 986

## Description

As expressed in the title of this specification, the present invention, refers to blood pumping equipment for extracorporeal circulation, whose purpose is to substitute the pumping action of the heart of a patient when the patient is subjected to heart surgery, it likewise being useful as a support means of ventricular assistance, respiratory assistance and cerebral percussion, without ruling outs its use in dialysis.

The purpose of the invention is to integrate in a single fungible unit the elements or means that are used in pumping blood for extracorporeal circulation, permitting the replacement of this unit or assembly after each operation, with a minimum loss of time, easy assem-bly/disassembly and with an economic cost much lower than that implied by systems or equipment used conventionally for the same purposes, eliminating the length of tubes and the volume of blood that would be involved. Basically, the equipment comprises a reservoir receiving the patient's venous blood; an impulsion pump of that blood; a heat exchanger for thermal conditioning of the blood and an oxygenator, all complemented with corresponding valves in order to form a single assembly or unit that permits a pulsatile blood flow to be provided with adjustable flow and frequency, by the doctor himself or controlled by a signal of the patient's electrocardiogram.

### BACKGROUND OF THE INVENTION

As is well known, heart surgery in extracorporeal circulation operates on a stopped heart, therefore it is necessary to temporarily substitute the functioning thereof by means of a system that externally pumps the blood, thus maintaining the blood perfusion to the brain and to the rest of the vital organs of the body.

Nowadays the main types of systems used in the pumping of blood are based on peristaltic or centrifugal pumps. Both systems provide a flow fixed by unit of time, therefore in order to increase the flow its speed must be increased, which leads to the continuous blood diverging very considerably from the natural pulsatile flow generated by the human heart.

Taking into account that the greater the disparity between the natural blood flow and the flow generated by the pumping system, the greater the physiological deterioration suffered by the patient, it is evident that it proves to be logical and desirable to achieve the greatest approximation between the extracorporeally pumped blood flow and the natural pulsatile flow.

Systems or equipment that efficiently carry out this function are unknown, irrespective of the fact that current systems are constituted by several parts that need to be coupled together before the operation, having high losses of charge, and the subsequent loss of time in the preparation, and, what is even more important, without the desired optimum results.

Besides, it must be taken into account that the equipment used is disposed of after each operation, which implies a high cost.

Patent US 5,770,149 discloses an extracorporeal blood oxygenation system according to the preamble of claim 1 comprising an integrated blood pump heat exchanger/membrane oxygenator assembly which is mountable upon, and engageable with, a separate motor/drive assembly. The blood pump of the system incorporates a novel axial/centrifugal impeller assembly. A single monitoring/control console may be utilised to control some or all the operational variables and parameters of the system. Additionally, the monitoring control console may incorporate a computer or microprocessor which is programmed to cause the system to operate in a predetermined manner, with appropriate changes or modifications of the system as required by the particular patient, or as dictated by the type of operative procedure which is being performed on the patient. The operation patterns are fixed and are not adapted to the operational variables and parameters of the system controlled.

### DESCRIPTION OF THE INVENTION

The equipment object of the invention, provided for the pumping of blood for extracorporeal circulation and ventricular assistance, constitutes a unit or assembly integrated with an operating principle that permits the programming of pumping flows and frequency in an independent manner, with the particularity that the impulsion pump may be controlled by a computer (according to the doctor's orders or as of a measured physiological parameter), which permits the obtainment of flow and pressure waves with characteristics close to those existing in normal physiological operation.

Therefore, it may be said that the equipment of the invention implies a structurally simple solution with absolute functional reliability, for the purpose for which it has been developed, maintaining its fungible characteristics.

In this sense, the equipment in question is constituted as of a pump with a bellows-type piston, which comprises a chamber in which the piston itself is arranged, piston integral to a through shaft, in a sealed manner, through a cover that closes the bottom base of the chamber, whose shaft is operated by means of a servomotor related to a suitable mechanism that transforms the angular alternate movement at the outlet of the servomotor into linear alternate movement that is applied to the shaft of the piston, so that the piston is displaced alternately in an axial manner in the inside of the chamber, in order to achieve in one direction of displacement the input of the patient's venous blood to the chamber, and to produce in the other direction of displacement the impulsion of the blood to the outside from said chamber for the oxygenation thereof.

In relation to the cited chamber established in the impulsion pump, the same is communicated on the one hand with a reservoir or tank to which the patient's venous blood accedes, reservoir that is placed above the chamber and integral to the body thereof. In the inside of the chamber, specifically in the area of passage or communication thereof with the reservoir, there is a float sealing valve, which due to its low density with regard to that of the blood is pushed by the blood towards the closing position, when the blood contained in the chamber is impelled by the piston (by means of expansion of the bellows) towards the outlet nozzle, while when the piston is displaced in the opposite direction (the bellows contracts), increasing the capacity of the chamber, the valve is separated from the passage of communication with the reservoir, opening said passage and permitting the access of the venous blood from the cited reservoir to the chamber itself.

Therefore, the chamber from which the pumping of the blood takes place is variable in amplitude, which permits the variation of the flow maintaining the frequency and vice versa, therefore said two parameters may be controlled independently in order to adapt them to the patient's or doctor's needs.

The body of the cited chamber is assembled, with ease of becoming independent, on a base with suitable displacement means, such as wheels, that allow easy transfer of the equipment from one place to another; locking and securing means of said assembly having been provided for between said two parts, with the particularity that the bottom one constituting the cited base carries the mechanism that transform the rotating movement of the servomotor into alternate linear movement on the shaft of the piston, the servomotor also being assembled on said base.

On the other hand, a ball-check valve that closes the passage by gravity has been provided for on the duct corresponding to the outlet nozzle of the chamber in which the impulsion pump is established, in such a way that the opening is carried out only when the blood is impelled from the pump. After said ball-check valve a heat exchanging device is inserted, as a thermal conditioning means of the blood and, connected to the latter, a oxygenating device, from which the oxygenated blood comes out through a nozzle, returning to the patient. Said thermal conditioning and oxygenating devices are integral to the block formed by the reservoir and body of the pump, in such a way that said components or elements, including the valves, form a fungible assembly or unit, easily assembled/disassembled with regard to the base.

The cited integration gives rise to a single fungible unit or assembly, and entails the elimination of the operative phases in order to carry out the coupling of all of the independent elements, intercommunicating them with tubes, which implies a saving of material, installation and final disassembly time, to which it is necessary to add the saving of volume of blood existing in the circuit outside of the patient, a fact which is of the utmost importance, aside from eliminating with this part of the effect of hemolysis that the cited tubes produce.

The incorporation of a programmable automaton, that governs the servomotor in order to control the movement of the bellows-type piston and consequently the flow and frequency of the pump, is also characteristic of the invention, the programming being carried out by means of a PC on the market, that makes the calculations and establishes the communication with the automaton that controls the servomotor, there also being a screen for direct control of said automaton, it being possible to achieve that the performance of the equipment is similar to that of human physiological behavior, with some pressure and flow curve diagrams similar to those of the behavior of the blood system, evidencing the similarity achieved in the parameters achieved with elasticity in frequency, volume, pressure and curve form.

The fact that the equipment may be provided with an autonomous operation system should be pointed out, by means of an electric feed battery, that permits the patient to be transported while some organ of his is being assisted by the proposed equipment, it also being provided for that the automaton and the equipment itself are connected to each other by means of a cable with an indefinite length, permitting the equipment to be located next to the patient or next to the surgeon, the automaton being able to be separated in a second plane.

Finally, the possibility of providing the equipment with means to adjust its operation tempo should be cited, means alternate to the automaton, for the purpose of permitting the use thereof without conventional electric feed.

### BRIEF DESCRIPTION OF THE FIGURES

In order to complement the description that is going to be made hereinafter and for the purpose of helping to provide a better understanding of the characteristics of the invention, the present specification is accompanied by a set of drawings on the basis of which the innovations and advantages of the blood pumping equipment for extracorporeal circulation made in accordance with the object of the invention, will be more easily understood.
Figure 1 is a schematic representation according to a general perspective of the equipment of the invention, as well as a block that corresponds to a simulator of the patient conveniently connected to the equipment.
Figure 2 shows a perspective view of the equipment object of the invention, wherein all of its parts can be seen.
Figure 3 is a raised view, with a sectioned part, of the same equipment represented in the preceding figure, allowing one to see the constitution of the impulsion pump, its coupling to the base and the means for transmitting force from the servomotor to the shaft of the bellows-type piston that forms part of the impulsion pump.

### DESCRIPTION OF THE PREFERRED EMBODIMENT

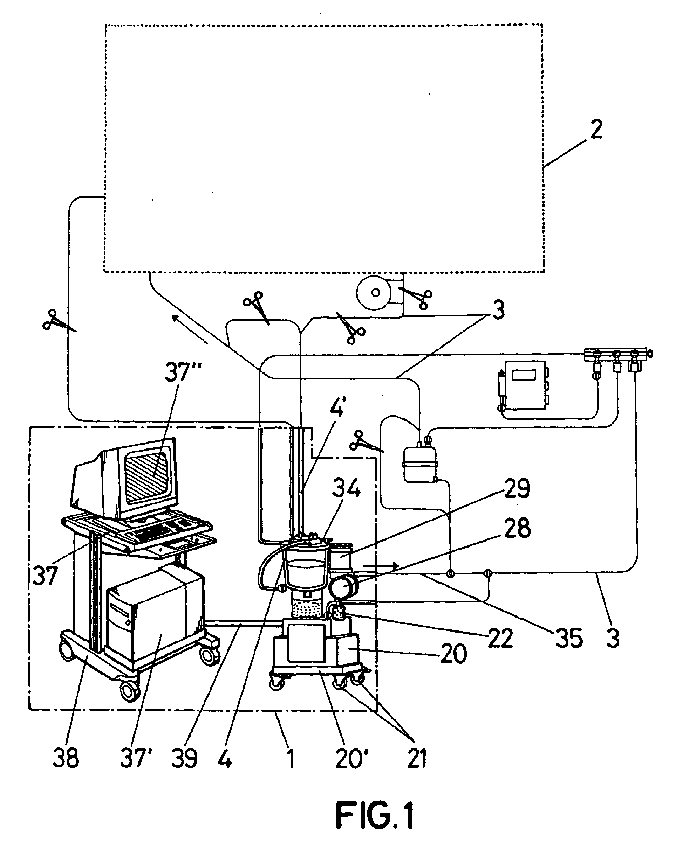

As can be seen in the cited figures, and specifically in connection with figure 1, it can be seen how the equipment of the invention, delimited by the contour generally referred to as number (1), is provided for its connection to a patient, whose simulator corresponds to the contour delimited by reference (2), this connection being established by means of tubes (3) and other devices and conventional components that do not have to be referred to as they are not object of the invention.

In relation to the equipment of the invention, the same comprises a reservoir (4) in the manner of a tank, provided with an inlet (4') to receive the venous blood coming from the patient. This reservoir (4) is coupled at the top on a body (5) constituting an impulsion pump, whose body (5) is defined by two parts, one corresponding to reference (5) itself and the other one corresponding to reference (5'), connected together by threading or by any other conventional system with the insertion of a sealing gasket (6), with the particularity that part (5') is the one that constitutes the coupling means for the top reservoir (4). A piston (7) with the capacity of axial displacement is assembled in the inside of body (5), and whose piston includes a bellows (8) and is integral to a shaft (9) guided on a bushing (10) provided on a bottom closing cover. The body (5-5') defines a chamber whose capacity is adjustable, depending on the position of the piston (7), in other words, on the greater or lesser extensibility of the bellows (8) belonging to said piston (7), extensibility and/or contraction that is achieved by means of upward and downward axial displacement of the shaft itself (9), that is operated from a servomotor (12) in which its shaft (13) is coupled to a mechanism (14) by means of which the rotating alternate movement of the shaft (13) belonging to the motor (12) is transformed into linear alternate movement of the shaft (9) corresponding to the piston (7).

The body (5-5') defines inside a chamber and as a whole an impulsion pump of the blood from the reservoir (4) towards an outlet nozzle (15), in such a way that the base hole for communication (16) between the reservoir (4) and the body chamber (5) or impulsion pump itself, may open and close by means of a float valve constituted by a low density sphere (17), that moves upward and downward between some side guides (18) that are curved at their bottom part in order to define an opening smaller than the diameter of the sphere (17), so that the sphere is maintained duly guided and positioned so that when it rises it carries out the closing of the passage or opening (16), against the corresponding sealing gasket (19) provided for therein.

The mechanism (14) that connects the shaft (9) of the piston (7) and the servomotor (12) is assembled on a frame (20) with respect to which the body (5-5') with the reservoir (4) may become independent, and whose frame (20) is in turn fastened on a displaceable base (20') provided with bottom wheels (21).

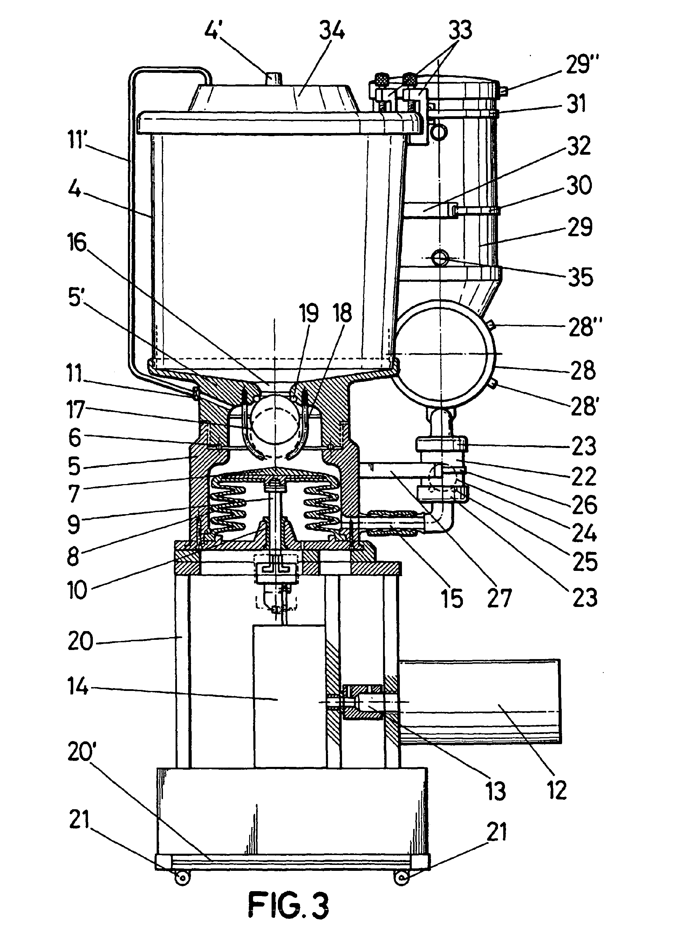

After the outlet nozzle (15) of the body (5-5') constituting the impulsion pump, a gravity ball-check valve has been provided for, a valve constituted as of a cylindrical hollow body (22) with respective top and bottom closing covers (23), and inside it there is a sphere (24) that tends, due to its weight, to close by gravity the inlet from the nozzle (15) to the inside of the body (22), carrying out the closing on the corresponding sealing gasket (25), as shown by the dash line in figure 3. This valve body (22) is integral, with a releasable nature, to the body (5) of the impulsion pump, by means of a brace (26) connected to a support (27) which in turn is integral to said body (5). After the ball-check valve (22) there is a heat exchanger (28), for thermal conditioning and then an oxygenator (29), these two elements forming a single body that is fastened to the reservoir (4) by means of braces (30) and (31), the brace (30) being fastened to a support (32) integral to the body itself of the reservoir (4), while the brace (31) is fastened to some clamps (33) that can be fastened on the top cover (34) of the reservoir itself (4). The oxygenator (29) is provided with the corresponding outlet (35) for the already oxygenated blood that will be returned to the patient himself.

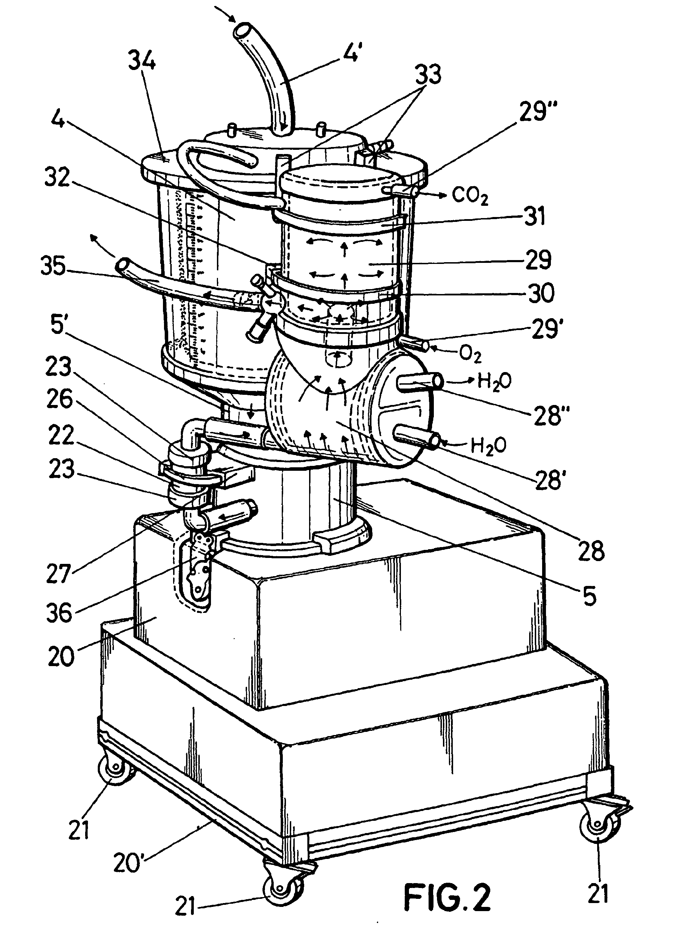

In the assembly of the body (5) of the impulsion pump on the bottom frame (20) a safety fastening (36) has been provided for, as shown in figure 2, wherein the inlet (28') of hot water to the heat exchanger (28) and the outlet (28'') of cold water from the exchanger (28).

Likewise, the oxygenator (29) includes an inlet (29') for oxygen and an outlet (29'') for carbon dioxide. The assembly that the reservoir (4), impulsion pump or body (5-5'), heat exchanger (28) and oxygenator (29), form with their corresponding valves as commented, constitute a disposable fungible unit that may be easily and rapidly assembled with regard to the frame (20), permitting the indefinite use thereof with its corresponding displaceable base (20') to which the above cited disposable unit may logically be coupled and decoupled, unit that constitutes the blood pumping equipment for extracorporeal circulation and ventricular assistance.

The operation is as follows:
When the servomotor (12) starts operating, the shaft (13) thereof turns alternately producing alternate movements, previously transformed into linear ones, on the shaft (9), which gives rise to axial displacement of the shaft in the inside of the chamber of the body (5), or that is to say extensibility and/or contraction of the bellows (8), giving rise in the first case to an impulsion of the blood contained in the chamber of the body (5) through the nozzle (15), in whose operating phase the low density sphere (17) is pushed upward pressing against the gasket (19) and therefore closing the passage or hole (16) for communication with the reservoir (4). In the second case, in other words when the bellows (8), by axial displacement downward of the shaft (9), contracts, then the sphere (17) is separated from the gasket (19) permitting the passage of blood through the hole (16) from the reservoir (4) to the chamber of the impulsion pump or body itself (5), all of this according to the alternate rotating movements of the servomotor (12), which can evidently be controlled and regulated at the will of the medical staff. In the impulsion of the pump, in other words, in the impulsion of the blood from the chamber of the body (5) through the nozzle (15), the opening of the valve (22) will be produced by pushing the sphere (24) upward, which will close the passage when there is no blood impulsion, closing that will be carried out by gravity as it has already been said.

It is noteworthy that as the reservoir (4) and the impulsion pump are conveniently associated, the aspiration of blood is carried out in negative pressure, really low, which benefits the conditions of preservation and oxygenation of the blood, very effectively avoiding the phenomenon of cavitation.

As it has also been stated, the servomotor (12) may also be controlled as far as its speed is concerned and to its angular rotation, controlling with it the displacements of the piston (7) and the pumping frequency, which is carried out by means of an automaton (37) that controls the different operating alternatives at the discretion of the doctor and/or the patient's measured physiological parameters, permitting the independent control of the pulse rate as well as of the blood flow. This automaton (37) is programmable by means of a PC (37') associated to the corresponding screen or monitor (37'') for direct control of the equipment.

Likewise, the equipment has means to purge the air of the top area of the pumping chamber, which is very important in order to prevent serious inconveniences that this accumulation of gas can produce in the operation of the equipment and/or patient, carrying out the priming prior to connection to the patient.

The cited purging means, as shown in figure 3, are formed by a purging valve (11) and the respective duct (11'') for return to the reservoir (4).

The assembly that has just been commented on is assembled on a rolling support (38), as shown in figure 1, and connected to the equipment of the invention by means of an interconnecting cable (39), with an indefinite length, for the purpose of permitting the use of the equipment at the desired distance with respect to the control automaton, permitting the automaton to be located anywhere and the equipment located next to the surgeon and/or patient.

Optionally, the equipment may be complemented with mechanical manually operated means that permit the same operation to be maintained in the event of a possible interruption in the feed of the electric fluid.

## Claims

1. Blood pumping equipment for extracorporeal circulation and ventricular assistance that comprises:
a reservoir (4) for receiving venous blood from the patient;
a pump (5) coupled under said reservoir (4);
said pump (5) comprised in a body (5-5') that comprises:
an internal chamber where the pumping means for the blood impulsion are arranged,
a first inlet that provides a passage (16) for communication of the reservoir (4) with the internal chamber,
a first outlet;
a heat exchanger (28) for thermal conditioning of the blood;
an oxygenator (29) arranged after the heat exchanger (28);
**characterized** that said pump (5) is driven by a piston (7) and in that said blood pumping equipment additionally comprises:
a nozzle (15) provided between the pump (5) outlet and the heat exchanger (28);
a first valve (17) provided for the first inlet,
said first valve (17) having:
a closed position to prevent the blood from flowing from the internal chamber to the reservoir (4) and
an open position to permit the blood from flowing from the reservoir (4) to the internal chamber;
a second valve (22) provided between the nozzle (15) and the heat exchanger (28);
said blood pumping equipment being connected to a computerized console (37) that controls the different operating alternatives of the blood pumping equipment controlling the piston (7) movement, said piston (7) affecting the free volume of the pump internal chamber, the pumping frequency and the acceleration and deceleration pattern in the blood admission and expulsion, at the discretion of the doctor and/or as of the measured physiological parameters of the patient, permitting the independent control of
the pulse rate,
the blood flow and
the wave form of the flow and pressure to generate a pulsing physiological flow by the pumping means acting in combination with the first valve (17)
permitting the aspiration by the pumping means when the first valve (17) is in the open position and
permitting the impulsion by the pumping means when the first valve (17) is in the closed position.

2. Blood pumping equipment for extracorporeal circulation and ventricular assistance, according to claim 1, **characterized in that** the blood impulsion pump is constituted by means of axial fastening of two bodies (5 and 5') between which a chamber is defined inside of which a piston (7) with bellows (8) is assembled, integral to a shaft (9) with alternate linear movement provided by a servomotor (12), between whose output shaft (13) and said shaft (11) of the piston of the bellows (8) a mechanism (14) has been provided for, mechanism that converts the rotating alternate movements of the servomotor (12) into linear alternate movements on the shaft (9) of the piston (7).

3. Blood pumping equipment for extracorporeal circulation and ventricular assistance, according to the preceding claims, **characterized in that** the valve (17) provided for in the hole or passage for communication (16) between the reservoir (4) and the chamber established in the body (5-5') of the impulsion pump, is constituted by a low density sphere that closes against an O-ring seal (19) provided for in the cited hole (16), a valve (17) that because of its low density is pushed by the weight itself of the blood of the reservoir (4) permitting the passage of the blood towards the chamber of the impulsion pump, in the situation of contraction of the bellows (8) of the piston (7), while when said bellows (8) expands, by upward displacement of the shaft (9), the impulsion of the blood from the chamber towards the outlet nozzle (15) is produced, the sphere (17) being pushed towards the closing position of the hole or passage (16) for communication with the reservoir (4); it being provided for that said valve (17) sphere is guided in its displacements by means of elements (18) that establish underneath a passage for the blood.

4. Blood pumping equipment for extracorporeal circulation and ventricular assistance, according to the preceding claims, **characterized in that** the reservoir (4), the body of the impulsion pump (5-5'), the valve (22), the heat exchanger (28) and the oxygenator (29) are preferably integrated in a single block.

5. Blood pumping equipment for extracorporeal circulation and ventricular assistance, according to claim 1, **characterized in that** the valve (22) provided after the outlet nozzle (15), is constituted by a tube-shaped body in vertical arrangement, inside of which there is a sphere (24) which by gravity carries out the closing of the bottom passage of said tube-shaped body, while the opening is carried out by means of the impelling force of the blood coming from the chamber established in the body (5-5') of the impulsion pump.

6. Blood pumping equipment for extracorporeal circulation and ventricular assistance, according to claim 4, **characterized in that** the tube-shaped body of the valve (22) is fastened to the body (5) of the impulsion pump, by means of a support (27) integral to the body and provided with a brace (26) that is adapted on the side surface of the tube-shaped body itself of the valve (22).

7. Blood pumping equipment for extracorporeal circulation and ventricular assistance, according to the preceding claims, **characterized in that** the heat exchanger (28), with its corresponding inlet for the blood, as well as the inlet (28') for hot water and the outlet (28'') for cold water, is integral to and communicated with the oxygenator (29), the oxygenator including an inlet (29') for oxygen, an outlet (29'') for carbon dioxide and an outlet (35) for the oxygenated blood, said oxygenator (29) being fastened in a removable manner to the body of the reservoir (4) by means of braces (30 and 31) connected to a support (32) and some clamps (33), respectively.

8. Blood pumping equipment for extracorporeal circulation and ventricular assistance, according to the preceding claims, **characterized in that** the computerized console (37) is connected to the fungible assembly or unit by means of an interconnecting cable (39) of variable length, making it possible to locate the fungible unit or assembly itself next to the patient and/or surgeon, keeping said console (37) at a distance, console which is programmed by means of a PC (37') connected to a monitor or screen (37'') that carries out the calculations and communication with the control automaton of the servomotor, all of this arranged on a displaceable support base (38).

9. Blood pumping equipment for extracorporeal circulation and ventricular assistance, according to the preceding claims, **characterized in that** it includes purging means in the top area of the pumping chamber, constituted preferably by a purging valve (11) located in an outlet of the top part of said pumping chamber, from which the corresponding duct (11') is projected, duct which ends in the top part of the reservoir (4).

## Patentansprüche

1. Blutpumpeinrichtung für Extrakorporalkreislauf und Ventrikulärunterstützung, die umfasst:
einen Vorratsbehälter (4) zum Aufnehmen von venösem Blut von dem Patienten;
eine Pumpe (5), die unter dem Vorratsbehälter (4) angebracht ist;
wobei die Pumpe (5) in einem Körper (5-5') enthalten ist, der umfasst
eine innere Kammer, in der die Pumpeeinrichtung zum Ausdrücken von Blut angeordnet ist,
einen ersten Einlass, der einen Kanal (16) zur Verbindung des Vorratsbehälters (4) mit der inneren Kammer bildet,
einen ersten Auslass;
einen Wärmetauscher (28) zur Wärmebehandlung des Blutes;
einen Oxygenator (29), der nach dem Wärmetauscher (28) angeordnet ist;
**dadurch gekennzeichnet, dass** die Pumpe (5) von einem Kolben (7) angetrieben wird und dass die Blutpumpeinrichtung zusätzlich umfasst:
eine Düse (15), die zwischen dem Auslass der Pumpe (5) und dem Wärmetauscher (28) vorhanden ist;
ein erstes Ventil (17), das für den ersten Einlass vorhanden ist, wobei das erste Ventil (17) hat
eine geschlossene Position, in der es verhindert, dass Blut aus der inneren Kammer in den Vorratsbehälter (4) fließt, und
eine offene Position, in der es zulässt, dass das Blut aus dem Vorratsbehälter (4) in die innere Kammer fließ4
ein zweites Ventil (22), das zwischen der Düse (15) und dem Wärmetauscher (28) vorhanden ist,
wobei die Blutpumpeinrichtung mit einem computergesteuerten Bedienpult (37) verbunden ist, das die verschiedenen Betriebsalternativen der Blutpumpeinrichtung durch Steuern der Bewegung des Kolbens (7) steuert, wobei der Kolben das freie Volumen der inneren Kammer der Pumpe, die Pumpfrequenz sowie das Muster der Beschleunigung und Verlangsamung des Einströmens und Ausstoßens von Blut beeinflusst, und zwar nach dem Ermessen des Arztes und/oder nach den gemessenen physiologischen Parametern des Patienten, wobei es unabhängige Steuerung
der Pulsfrequenz,
des Blutstroms und
der Wellenform des Stroms und Drucks zulässt,
um einen pulsierenden physiologischen Strom durch die Pumpeinrichtung zu erzeugen, die in Kombination mit dem ersten Ventil (17) wirkt, und
das Ansaugen durch die Pumpeinrichtung zuzulassen, wenn sich das erste Ventil (17) in der offenen Position befindet, und das Ausdrücken durch die Pumpeinrichtung zuzulassen, wenn sich das erste Ventil (17) in der geschlossenen Position befindet.

2. Blutpumpeinrichtung für Extrakorporalkreislauf und Ventrikulärunterstützung nach Anspruch 1, **dadurch gekennzeichnet, dass** die Blutausdrückpumpe durch axiale Befestigung von zwei Körpern (5 und 5') gebildet wird, zwischen denen eine Kammer begrenzt ist, in der ein Kolben (7) mit Balg (8) integral mit einer Welle (9) mit abwechselnder linearer Bewegung angebracht ist, die durch einen Servomotor (12) erzeugt wird, wobei zwischen seiner Ausgangswelle (13) und der Welle (11) des Kolbens des Balgs (8) ein Mechanismus (14) vorhanden ist und der Mechanismus die abwechselnden Drehbewegungen des Servomotors (12) in abwechselnde lineare Bewegungen an der Welle (9) des Kolbens (7) umwandelt.

3. Blutpumpeinrichtung für Extrakorporalkreislauf und Ventrikulärunterstützung nach den vorangehenden Ansprüchen, **dadurch gekennzeichnet, dass** das Ventil (17), das in dem Loch oder dem Kanal zur Verbindung (16) zwischen dem Vorratsbehälter (4) und der in dem Körper (5-6') der Ausdrückpumpe ausgebildeten Kammer vorhanden ist, durch eine Kugel geringer Dichte gebildet wird, die an einer O-Ring-Dichtung (19) abschließt, die in dem Loch (16) vorhanden ist, wobei das Ventil (17) aufgrund seiner geringen Dichte durch das Gewicht des Blutes des Vorratsbehälters (4) selbst nach unten gedrückt wird und das Hindurchtreten des Blutes in Richtung der Kammer der Ausdrückpumpe zulässt, wenn der Balg (8) des Kolbens (7) eingezogen wird, während, wenn sich der Balg durch Verschiebung der Welle (9) nach oben ausdehnt, Blut aus der Kammer in Richtung der Auslassdüse (15) ausgedrückt wird, wobei die Kugel (17) in Richtung der Schließposition des Lochs bzw. des Kanals (16) zur Verbindung mit dem Vorratsbehälter (4) gedrückt wird, und vorgesehen ist, dass die Kugel des Ventils (17) bei ihren Verschiebungen von Elementen (18) geführt wird, die darunter einen Kanal für das Blut bilden.

4. Blutpumpeinrichtung für Extrakorporalkreislauf und Ventrikulärunterstützung nach den vorangehenden Ansprüche, **dadurch gekennzeichnet, dass** der Vorratsbehälter (4), der Körper der Förderpumpe (5-5'), das Ventil (22), der Wärmetauscher (28) und der Oxygenator (29) vorzugsweise in einen einzelnen Block integriert sind.

5. Blutpumpeinrichtung für Extrakorporalkreislauf und Ventrikulärunterstützung nach Anspruch 1, **dadurch gekennzeichnet, dass** das Ventil (22), das nach der Auslassdüse (15) vorhanden ist, durch einen röhrenförmigen Körper in vertikaler Anordnung gebildet wird, in dem sich eine Kugel (24) befindet, die durch Schwerkraft das Verschließen des unteren Durchlasses des röhrenförmigen Körpers ausführt, während das Öffnen durch die treibende Kraft des Blutes ausgeführt wird, das aus der Kammer kommt, die in dem Körper (5-5') der Ausdrückpumpe ausgebildet ist.

6. Blutpumpeinrichtung für Extrakorporalkreislauf und Ventrikulärunterstützung nach Anspruch 4, **dadurch gekennzeichnet, dass** der röhrenförmige Körper des Ventils (22) an dem Körper (5) der Förderpumpe mittels eines Trägers (27) befestigt ist, der integral mit dem Körper ausgebildet ist und mit einer Klammer (26) versehen ist, die an der Seitenfläche des röhrenförmigen Körpers des Ventils (22) angeordnet ist.

7. Blutpumpeinrichtung für Extrakorporalkreislauf und Ventrikulärunterstützung nach den vorangehenden Ansprüchen, **dadurch gekennzeichnet, dass** der Wärmetauscher (28) mit seinem entsprechenden Einlass für das Blut sowie mit dem Einlass (28') für warmes Wasser und dem Auslass (28") für kaltes Wasser integral mit dem Oxygenator (29) ausgebildet ist und mit ihm in Verbindung steht, wobei der Oxygenator einen Einlass (29') für Sauerstoff, einen Auslass (29") für Kohlendioxid und einen Auslass (35) für das mit Sauerstoff angereicherte Blut enthält und der Oxygenator (29) abnehmbar an dem Körper des Vorratsbehälters (24) mit Klammern (30 und 31) befestigt ist, die mit einem Träger (32) bzw. Klemmen (33) verbunden sind.

8. Blutpumpeinrichtung für Extrakorporalkreislauf und Ventrikulärunterstützung nach den vorangehenden Ansprüchen, **dadurch gekennzeichnet, dass** das computergesteuerte Bedienpult (37) mit der beweglichen Anordnung oder Einheit mittels eines Verbindungskabels (39) veränderlicher Länge verbunden ist, wodurch es möglich wird, die bewegliche Einheit oder Anordnung neben dem Patienten und/oder Chirurgen zu positionieren und das Bedienpult (37) in einem Abstand zu halten, wobei das Bedienpult mittels eines PC (37') programmiert wird, der mit einem Monitor oder Bildschirm (37") verbunden ist und der die Berechnungen sowie Kommunikation mit dem Steuerautomaten des Servomotors ausführt, wobei alle diese Einrichtungen auf einem verschiebbaren Trageuntersatz (38) angeordnet sind.

9. Blutpumpeinrichtung für Extrakorporalkreislauf und Ventrikulärunterstützung nach den vorangehenden Ansprüchen, **dadurch gekennzeichnet, dass** sie eine Spüleinrichtung in dem oberen Bereich der Pumpenkammer enthält, die vorzugsweise durch ein Spülventil (11) gebildet wird, das sich an einem Auslass des oberen Teils der Pumpekammer befindet, von dem die entsprechende Leitung (11') vorsteht, wobei die Leitung im oberen Teil des Vorratsbehälters (4) endet.

## Revendications

1. Equipement de pompage du sang pour circulation extracorporelle et assistance ventriculaire comprenant :
un réservoir (4) destiné à recevoir le sang veineux provenant du patient ;
une pompe (5) couplée sous ledit réservoir (4) ;
ladite pompe (5) étant comprise dans un corps (5-5') comprenant :
une chambre interne dans laquelle est disposé le moyen de pompage pour l'impulsion sanguine,
un premier orifice d'entrée fournissant un passage (16) permettant la communication du réservoir (4) avec la chambre interne,
un premier orifice de sortie
un échangeur de chaleur (28) pour le conditionnement thermique du sang ;
un oxygénateur (29) disposé en aval de l'échangeur de chaleur (28) ;
**caractérisé en ce que** ladite pompe (5) est entraînée par un piston (7) et **en ce que** ledit équipement de pompage du sang comprend en outre :
une buse (15) prévue entre l'orifice de sortie de la pompe (5) et l'échangeur de chaleur (28) ;
une première vanne (17) prévue pour le premier orifice d'entrée, ladite première vanne (17) présentant :
une position fermée pour empêcher l'écoulement du sang de la chambre interne vers le réservoir (4) et
une position ouverte pour permettre l'écoulement du sang du réservoir (4) vers la chambre interne ;
une seconde vanne (22) prévue entre la buse (15) et l'échangeur de chaleur (28) ;
ledit équipement de pompage du sang étant relié à une console informatisée (37) commandant les différentes possibilités de fonctionnement de l'équipement de pompage du sang en commandant le déplacement du piston (7), ledit piston (7) modifiant le volume libre de la chambre interne de la pompe, la fréquence de pompage et le schéma d'accélération et de décélération de l'admission et de l'expulsion de sang, à la discrétion du docteur et/ou selon les mesures des paramètres physiologiques du patient, en permettant la commande indépendante
du pouls,
du flux sanguin et
de la forme d'onde du flux et de la pression
pour générer un flux physiologique pulsé grâce au moyen de pompage qui agit en combinaison avec la première vanne (17),
en permettant au moyen de pompage d'effectuer l'aspiration lorsque la première vanne (17) est dans la position ouverte et
en permettant au moyen de pompage d'effectuer l'impulsion lorsque la première vanne (17) est dans la position fermée.

2. Equipement de pompage du sang pour circulation extracorporelle et assistance ventriculaire selon la revendication 1, **caractérisé en ce que** la pompe pour l'impulsion sanguine est constituée par la fixation axiale de deux corps (5 et 5') entre lesquels est définie une chambre dans laquelle un piston (7) à soufflet (8) est assemblé, d'un seul tenant avec un arbre (9) à mouvement linéaire alternatif fourni par un servomoteur (12), un mécanisme (14) ayant été prévu entre l'arbre de sortie (13) dudit servomoteur et ledit arbre (11) du piston à soufflet (8), lequel mécanisme convertit les mouvements de rotation alternatifs du servomoteur (12) en mouvements linéaires alternatifs sur l'arbre (9) du piston (7).

3. Equipement de pompage du sang pour circulation extracorporelle et assistance ventriculaire selon les revendications précédentes, **caractérisé en ce que** la vanne (17) prévue dans le trou ou passage de communication (16) entre le réservoir (4) et la chambre établie dans le corps (5-5') de la pompe à impulsion est constituée d'une sphère à basse densité qui se ferme contre un joint torique (19) prévu dans ledit trou (16), une vanne (17) qui en raison de sa basse densité est poussée par le poids même du sang du réservoir (4) en permettant le passage du sang vers la chambre de la pompe à impulsion, dans la situation de contraction du soufflet (8) du piston (7), tandis que lorsque ledit soufflet (8) se relâche, du fait de la montée de l'arbre (9), l'impulsion du sang a lieu depuis la chambre vers la buse de sortie (15), la sphère (17) étant poussée vers la position fermée du trou ou passage (16) de communication avec le réservoir (4) ; en prévoyant que ladite vanne (17) soit guidée dans ses déplacements au moyen d'éléments (18) qui établissent par-dessous un passage pour le sang.

4. Equipement de pompage du sang pour circulation extracorporelle et assistance ventriculaire selon les revendications précédentes, **caractérisé en ce que** le réservoir (4), le corps de la pompe à impulsion (5-5'), la vanne (22), l'échangeur de chaleur (28) et l'oxygénateur (29) sont de préférence intégrés dans un seul bloc.

5. Equipement de pompage du sang pour circulation extracorporelle et assistance ventriculaire selon la revendication 1, **caractérisé en ce que** la vanne (22) prévue en aval de la buse de sortie (15) est constituée d'un corps tubulaire agencé verticalement, à l'intérieur duquel se trouve une sphère (24) qui effectue par gravité la fermeture du passage inférieur dudit corps tubulaire, tandis que l'ouverture se produit au moyen de la force d'impulsion du sang provenant de la chambre établie dans le corps (5-5') de la pompe à impulsion.

6. Equipement de pompage du sang pour circulation extracorporelle et assistance ventriculaire selon la revendication 4, **caractérisé en ce que** le corps tubulaire de la vanne (22) est fixé au corps (5) de la pompe à impulsion au moyen d'un support (27) formant une seule pièce avec le corps et muni d'une bride (26) qui est adaptée sur la surface latérale du corps tubulaire de la vanne (22).

7. Equipement de pompage du sang pour circulation extracorporelle et assistance ventriculaire selon les revendications précédentes, **caractérisé en ce que** l'échangeur de chaleur (28), avec son orifice d'entrée de sang correspondant, ainsi que l'orifice d'entrée (28') d'eau chaude et l'orifice de sortie (28") d'eau froide, forme une seule pièce avec l'oxygénateur (29) et communique avec celui-ci, l'oxygénateur comprenant un orifice d'entrée (29') pour l'oxygène, un orifice de sortie (29") pour le dioxyde de carbone et un orifice de sortie (35) pour le sang oxygéné, ledit oxygénateur (29) étant fixé de façon amovible au corps du réservoir (4) au moyen de brides (30 et 31) raccordées à un support (32) et de quelques brides de serrage (33), respectivement.

8. Equipement de pompage du sang pour circulation extracorporelle et assistance ventriculaire selon les revendications précédentes, **caractérisé en ce que** la console informatisée (37) est reliée au groupe ou unité fongible au moyen d'un câble d'interconnexion (39) de longueur variable, ce qui permet de disposer l'unité ou groupe fongible à proximité du patient et/ou du chirurgien, en maintenant ladite console (37) à une certaine distance, laquelle console est programmée au moyen d'un PC (37'), relié à un moniteur ou écran (37"), qui effectue les calculs et la communication avec l'automate de commande du servomoteur, tous ces éléments étant agencés sur une base de support déplaçable (38).

9. Equipement de pompage du sang pour circulation extracorporelle et assistance ventriculaire selon les revendications précédentes, **caractérisé en ce qu'**il comprend un moyen de purge dans la zone supérieure de la chambre de pompage, constituée de préférence d'une vanne de purge (11) située dans un orifice de sortie de la partie supérieure de ladite chambre de pompage, à partir de laquelle le conduit (11') correspondant s'étend en se terminant dans la partie supérieure du réservoir (4).
